# EUROPEAN PATENT APPLICATION

(11) **EP 1 917 854 A1**
(43) Date of publication of application: **07.05.2008**
(21) Application number: 07119593.7
(22) Date of filing: 30.10.2007
(51) Int. Cl.: A01K 67/027

(54) **Method for producing anti idiotypic antibodies**

(30) Priority: 06.11.2006 EP 06123503
(71) Applicant: F. Hoffmann-La Roche AG, 4070 Basel (CH)
(72) Inventor: Beck, Hermann, 79539, Loerrach (DE); Iglesias, Antonio, 79102, Freiburg (DE); Schreitmueller, Thomas, 4147, Aesch BL (CH); Zocher, Marcel, 79539, Loerrach (DE)
(74) Representative: Küng, Peter

(57) **Abstract**

The present invention relates to a use of a non-human animal transgenic for an exogenous antibody for generating anti-idiotypic antibodies and a method for generating anti-idiotypic antibodies comprising a) creating a non-human animal transgenic for an exogenous antibody, b) inducing an immune response in said transgenic non-human animal against an antibody of interest, whereby the antibody of interest comprises the same species-specific isotype as the exogenous antibody, and c) generating antibodies directed against the idiotypic part of the antibody of interest.

## Description

The use of monoclonal antibody (MAb) therapeutics in the treatment of cancer, autoimmune diseases, transplant rejection and other indications has experienced an important expansion in the recent years. In particular, molecular engineering has enabled the conversion of murine MAbs into humanized or totally human molecules, thus reducing the unwanted immune response against these therapeutic agents in treated patients (human anti-human antibodies = HAHA). As a consequence, the success of therapy with therapeutic MAbs has increased dramatically, mainly due to diminished or absent immunogenicity, increased serum half-life and improved effector functions. In this context, however, it has become crucial to develop appropriate assays apt to discriminate between therapeutic immunoglobulins (IgG) from the large pool of endogenous IgG molecules in the serum of treated patients. Hence, tools are needed for the analysis of pharmacokinetics, pharmacodynamics, biodistribution and the induction of immune responses to clinically administered therapeutic MAbs. Anti-idiotypic (anti-ID) antibodies bind specifically the variable region of other antibodies and therefore can be used to detect therapeutic MAbs in pharmacokinetic studies and help to quantify human-anti-human antibody (HAHA) responses in treated individuals. Currently, such anti-idiotypic antibodies are generated by immunization of experimental animals with the therapeutic antibody of interest, followed by screening for the presence of idiotypic-binding MAbs. Unfortunately, immune responses elicited in experimental animals with therapeutic MAbs are directed predominantly against the Fc portion of human antibodies, such that anti-idiotypic antibodies are rare and difficult to obtain. Furthermore, the generation of anti-idiotypic antibodies from animals by polyclonal serum affinity purification methods, e.g. high percentage of non-idiotypic binding antibodies, low yield of immuno-adsorption methods with normal immunoglobulin, and differing qualities of preparations from batch-to-batch.

Therefore, the present invention provides a use of a transgenic non-human animal for generating anti-idiotypic antibodies against an antibody of interest, wherein said non-human animal is transgenic for an exogenous antibody and wherein the antibody of interest has the same isotype as the exogenous antibody.

Such an animal transgenic for an exogenous antibody conveys tolerance to the Fc part of all antibodies of the same isotype as the exogenous antibody, while allowing an immune response to the variable regions of antibodies of the same isotype as the exogenous antibody but a different idiotype.

In EP 05105946.7 a non-human animal transgenic for an exogenous antibody was already described. However, the use as described herein was not disclosed.

In particular, the present invention provides a method for generating anti-idiotypic antibodies comprising:
a) creating a non-human animal transgenic for an exogenous antibody
b) inducing an immune response by said transgenic non-human animal against an antibody of interest, whereby the antibody of interest has the same species-specific isotype as the exogenous antibody, and
c) thereby generating antibodies directed against the idiotypic part of the antibody of interest.

Preferably, the method further comprises the additional step d) isolating the anti-idiotypic antibodies. Methods for isolating antibodies, in particular for isolating antibodies from body fluid (e.g. blood) are known in the art. More preferably, the anti-idiotypic antibodies are purified. Appropriate purifying methods are known to the skilled in the art.

The term "exogenous antibody" as used herein refers to an antibody comprising constant regions which originate from a source organism (e.g. human) and the DNA encoding said antibody has been introduced into a host organism (e.g. mouse) so that the host organism expresses that antibody. The source organism and the host organism do not belong to the same species according to the Linnaean taxonomic system. A species is a set of actually or potentially interbreeding populations.

The exogenous antibody may be a therapeutic antibody. Preferably, the exogenous antibody is a human, humanized or chimeric antibody, whereby at least the constant region of the chimera is human. More preferably, the exogenous antibody is an immunoglobulin gamma (IgG). Even more preferably, the antibody is the antibody against the human amyloid beta peptide or a variant thereof. The most preferred antibody is anti-Abeta IgG1. The anti-Abeta IgG1 is described in detail in the patent application WO 03/070760, which is herewith fully incorporated as reference.

The term "variant" or "antibody variant" as used herein refers to an antibody that differs in structural characteristics, the preparation method, formulation, or storage conditions from a standard antibody. The structural variants may comprise variation of the primary, secondary and tertiary protein structure (i.e. conformational changes), glycosylation and chemical modifications of amino acids. Further structural variations are i.e. alternative inter-domain constructs (VL/VL, VH/VH), dimers, oligomers and larger aggregates.

The term "antibody of interest" as used herein refers to an antibody whose constant regions belong to the same isotype as the constant regions of the exogenous antibody.

An antibody is a "Y"-shaped molecule and consists of two heavy chains and two light chains. There are different types and subtypes of both, the heavy chains and the light chains. Each heavy chain and each light chain has a constant region and a variable region, whereby the size of constant regions is bigger for the heavy chain.

The term "constant region" or "C region" refers to a region of an antibody molecule that is nearly identical with the corresponding regions of antibodies of different specificities produced by organisms of the same species. The constant part is invariable within the same antibody class (isotype) and is responsible for the effector functions of a particular Immunoglobulin subclass. An Fc fragment refers to a fragment of an antibody generated by the cleavage of an antibody with the enzyme papain and comprising the most part of the constant regions.

The term "variable region" as used herein refers to a region of an antibody molecule which binds to specific antigens. It is composed of the combination of the antigen-binding sites of the heavy and of the light chain. It differs between immunoglobulins of different B cells, but is the same for all immunoglobulins produced by the same B cell. The variable region is generated somatically via a gene recombination process taking place during maturation of B-cells. It is this process of rearrangement that creates the enormous diversity needed to bind any given antigen, and that thus enables the immune system to recognize and neutralize the innumerable antigenic burdens posed by foreign and pathogenic structures. As a consequence, the antibody pool is composed of immunoglobulins bearing a large repertoire of different V regions, while sharing the same Fc portions.

The term "fragment binding antigen" or "Fab fragment" is an antibody fragment that includes the variable, antigen-binding region, and which is generated by the cleavage of an antibody with the enzyme papain.

The term "idiotypic region" as used herein refers to the part of the variable region of an antibody that is unique for each antibody type.

The term "anti-idiotypic antibody" as used herein refers to an antibody which binds to the idiotypic region of another antibody.

The term "immune response" as used herein refers to a response of the immune system to the presence of an antigen. It involves the production of antibodies capable of binding to the antigen.

The term "isotype" refers to antigenic determinants that characterize classes and subclasses of heavy chains and types and subtypes of light chains. Antibodies of different isotypes have not only different variable regions but also differences in the constant regions. Therefore, for example IgG and IgM antibodies of the same species belong to different isotypes. An antibody of one species introduced into another species will preferentially induce generation of anti-xenogeneic antibodies mostly directed to immunogenic region in the isotype (isotypic determinants).

A preferred embodiment of the invention is a method of generating anti-idiotypic antibodies against a human therapeutic antibody, comprising
a) creating a non-human animal transgenic for a human IgG antibody
b) inducing an immune response against said therapeutic antibody in said transgenic animal, and
c) generating anti-idiotypic antibodies specific for the idiotypic part of the therapeutic antibody.

Preferably, the method further comprises the additional step d) isolating the anti-idiotypic antibodies.

The transgenic non-human animal may be any non-human animal. The preferred non-human animal is a mammal. More preferably, the non-human animal is a rodent such as a mouse or a rat. Methods for producing a transgenic non-human animal are well known in the art. Suitable methods are described i.e. in Hogan B., Beddington R., Costantini F. & Lacy E. Manipulating the mouse embryo. A laboratory manual. 2nd Edition (1994). Cold Spring Harbor Laboratory Press.

A preferred method of producing a non-human transgenic animal expressing an exogenous antibody comprises
a) introducing a genetic construct comprising the DNA encoding the exogenous antibody into a non-human zygote or an non-human embryonic stem cell,
b) generating a transgenic non-human animal from said zygote or embryonic stem cell, and thereby,
c) producing a transgenic non-human animal expressing the exogenous antibody.

For example, the transgenic animals may be generated by injecting above described DNA construct into the pronucleus of zygotes, transferring these injected zygotes into pseudo-pregnant foster mothers, breeding founder animals resulting from the oocytes to wild type animals, testing the offspring resulting from these breedings for the presence of the synthetic DNA transgene construct, breeding hemizygous animals, optionally generating homozygous transgenic animals.

Alternatively, the transgenic animals may be generated by introducing the genetic construct as described above into embryonic stem cells and subsequently selecting embryonic stem cell clones for the presence of the transgene in the genome, verifying the presence of the transgene in the transformed embryonic stem cell clones, injecting the verified recombinant embryonic stem cells into blastocysts of wild type animals, transferring these injected blastocysts into pseudo-pregnant foster mothers, breeding chimeras resulting from the blastocysts to wild type animals, testing the offspring resulting from these breedings for the presence of the transgene, breeding hemizygous animals, optionally generating homozygous transgenic animals.

A non-human transgenic animal as used in the invention may also be a progeny of a non-human transgenic animal produced by one of the above described methods. A progeny may be obtained by breeding said non-human transgenic animal, whereby said progeny retains the same phenotype as said transgenic animal.

The zygote or embryonic stem cell may derive from any non-human animal. Preferably, the zygote or embryonic stem cell derives from a rodent. More preferably, the zygote or embryonic stem cell derives from a mouse.

For creating a transgenic mouse, the zygote or embryonic stem cells comprise, but are not limited to, zygotes or embryonic stem cells derived from C57BL/6J, CBA/, BALB/c, DBA/2 and SV129 (Seong, E et al (2004) Trends Genet. 20, 59-62; Wolfer, D.P. et al., Trends Neurosci. 25 (2002): 336-340).

The expression of the antibody in the transgenic non-human animal may be constitutive or inducible. Preferably, the expression of the antibody is constitutive.

Inducing an immune response of an animal against an antibody may be done by methods comprising, for example, injecting said antigen subcutaneously, intravenously, intralesionaly, intramuscularly or intraperitoneally (i.p.) or by administration of the antigen orally (p.o.).

For treatment of the transgenic non-human animal, the antibody of interest may be diluted or emulsified in a pharmaceutically acceptable carrier suitable for administration to a transgenic non-human animal. The carrier is a liquid carrier. Suitable carriers are well known to the person skilled in the art, such as for example, a saline solution. Preferably, the carrier is Rehydragel-HPA.

Method for isolating the generated anti-idiotypic antibodies are well known to the skilled person in the art. A preferred method comprises a) obtaining a blood sample from the immunized non-human animal transgenic for an exogenous antibody, b) preparing serum, for example by coagulation, and c) separating and purifying anti-idiotypic antibodies by chromatographic methods such as, for example, affinity chromatography, ion exchange chromatography and/or size exclusion chromatography.

The non-human transgenic animal as described herein may also be used for producing ant-idiotypic monoclonal antibodies against an antibody of interest. Methods for producing monoclonal methods are well known to the person skilled in the art. Such a method may, for example, be a method comprising a) isolation of spleen cells of the non-human animal transgenic for an exogenous antibody, b) preparing myeloma cells, and c) fusing the spleen cells with the myeloma cells. The so created hybridoma cells produce monoclonal anti-idiotypic antibodies.

The transgenic animal expressing an exogenous antibody acquires immunological tolerance to this particular antbody. A defined transgenic antibody, such as for example human anti-Abeta IgG1, conveys tolerance to the Fc part of all antibodies of the same isotype, while allowing an immune response to V regions of other antibodies.

The method of the invention may be used for generating antibodies specifically recognizing therapeutic antibodies. Such anti-idiotypic antibodies are valuable in pharmacokinetic studies as well as in studies of clinical human-anti-human antibody (HAHA) responses in individuals treated with the corresponding therapeutic monoclonal antibodies.

Furthermore, anti-idiotypic antibodies generated with the transgenic mouse as described above may mimic antigenic determinants and may thus serve as surrogate antigens for diagnostic purposes, e.g. for applications where the availability of antigen is limiting. Applications include competitive immunassays or direct serological assays. Advantages of using "internal image" anti-idiotypic antibodies instead of conventional antigens include ease of production, safety of use in cases where the antigen is toxic or hazardous for other reasons, ease of purification, established methods for the attachment of label, and possibilities for the attachment to a solid support without loss of immunoreactivity.

Many autoimmune diseases are characterized by the appearance of auto-antibodies. Following identification of the idiotype carried by the auto-antibody, the described transgenic mouse may be useful for the generation of anti-idiotypic antibodies for the diagnosis of antibody-mediated and autoantibody-accompanied autoimmune disease. Elevated expression of specific IDs has been demonstrated in diseases such as myasthenia gravis, Hashimoto's thyroiditis, rheumatoid arthritis and systemic lupus erythromatosus (Isenberg DA, Williams W, Axford J, et al., "Comparison of Anti-DNA Antibody Idiotypes in Human Sera," J Autoimmunity, 3:393-414, 1990). Use as surrogate antigens for diagnosis of human infectious disease is yet another useful application of anti-idiotypic antibodies.

Having now generally described this invention, the same will become better understood by reference to the specific examples, which are included herein for purpose of illustration only and are not intended to be limiting unless otherwise specified, in connection with the following figures.

### Figures:

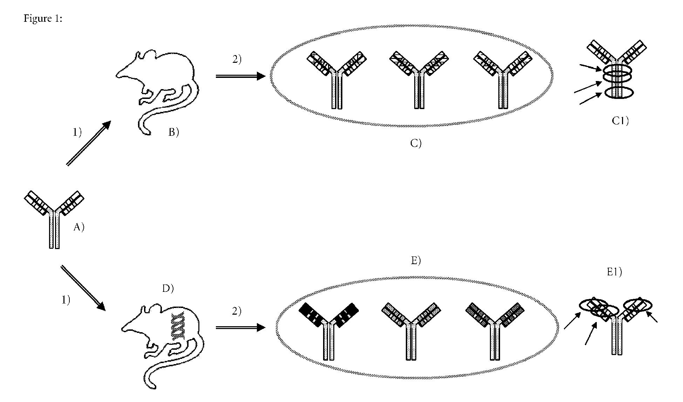

Figure 1 shows a schematic overview of the methodology. A wildtype mouse immunized with e.g. a monoclonal human IgG antibody will produce antibodies mostly directed to the isotypic part of the monoclonal antibody. These antibodies cross-recognize all human IgGs. A mouse transgenic for the monoclonal human IgG antibody which is immunized with e.g. a monoclonal human IgG antibody, produces antibodies directed primarily to the variable part of the immunized IgG (idiotypic part of the antibody), thus lower crossrecognition with other human IgGs. 1) = immunization; 2) = immune response; **A**) = monoclonal human IgG antibody; **B**) Wildtype mouse, **C**) = produced antibodies mainly directed to isotypic part of the monoclonal antibody (A), **C1**) = recognition sites of the produced antibodies (C) are located in the isotypic part of the monoclonal human antibdody (A); **D**) mouse transgenic for monoclonal human IgG antibody (A); **E**) = produced antibodies mainly directed to idiotypic part of the monoclonal antibody (A), **E1**) = recognition sites of the produced antibodies (E) are located in the idiotypic part of the monoclonal human antibdody (A).

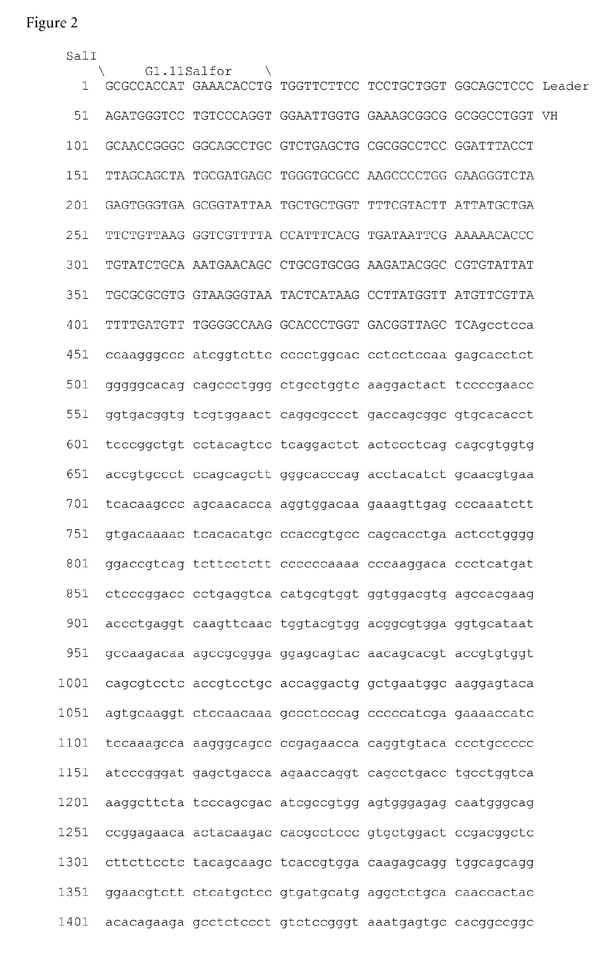

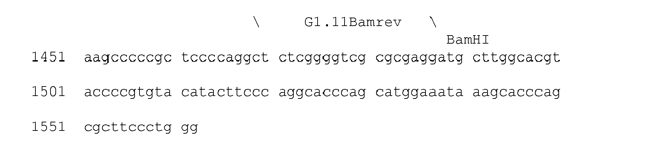

Figure 2 shows the coding sequence of the human Ig γ1 gene specific for human Aβ as cloned into the expression vector pHSE3' for transgenic expression. The position and the name of the primers used in PCR amplification are displayed above or below the corresponding sequence. The leader sequence is shown in italics. The stop codon is shown in bold.

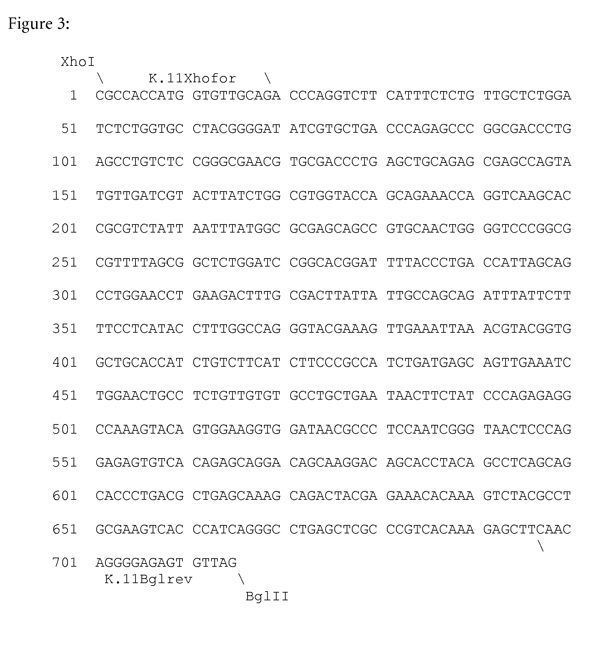

Figure 3 shows the coding sequence of the human Ig κ gene specific for human Aβ as cloned into the expression vector pHSE3' for transgenic expression. The position and the name of the primers used in PCR amplification are displayed above or below the corresponding sequence. The leader sequence is shown in italics. The stop codon is shown in bold.

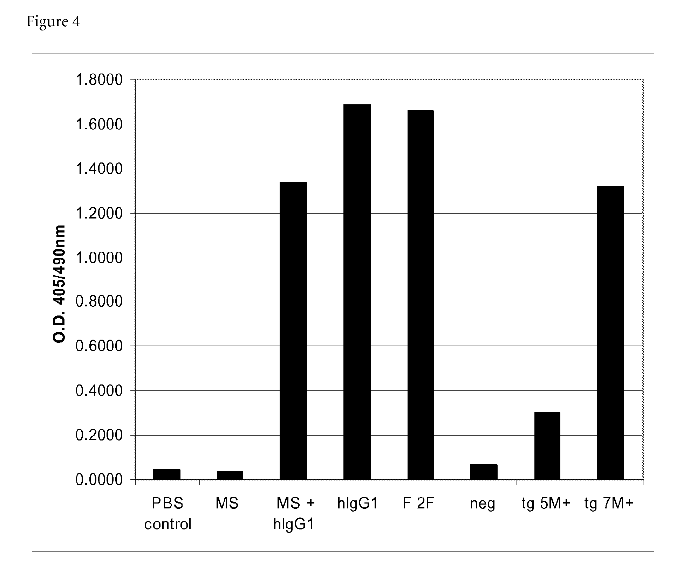

Figure 4 shows a graphical representation of a serum analysis of mice transgenic transgenic for anti-Abeta IgG1 (= Mab-11). Sandwich ELISA specific for human kappa light chain and human gamma heavy chain was performed. MS: mouse serum. hIgG1: recombinant human immunoglobulin of gamma 1 isotype. F 2F: founder mouse 2F. Neg: PCR-negative littermate control. Tg 5M+: transgenic mouse 5M+. Tg 7M+: transgenic mouse 7M+. Transgenic mice expressed both antibody chains within the same molecule.

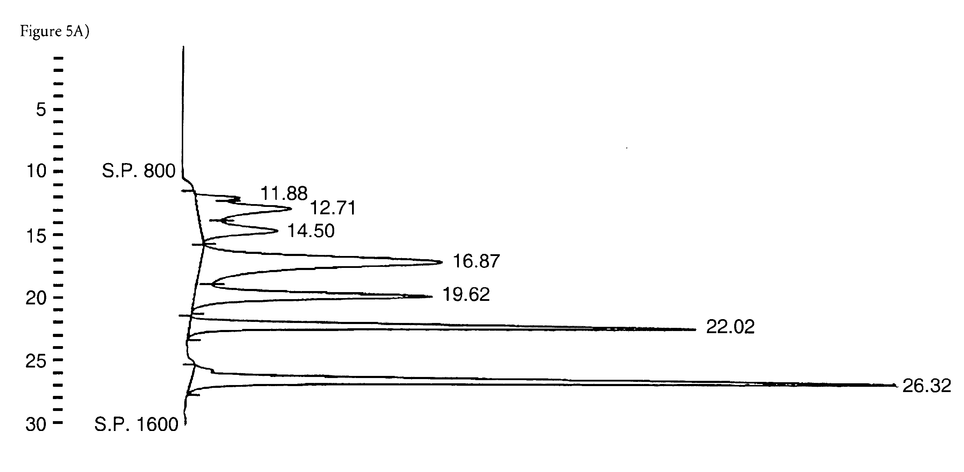

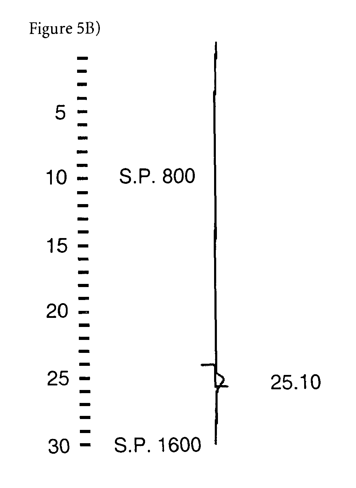

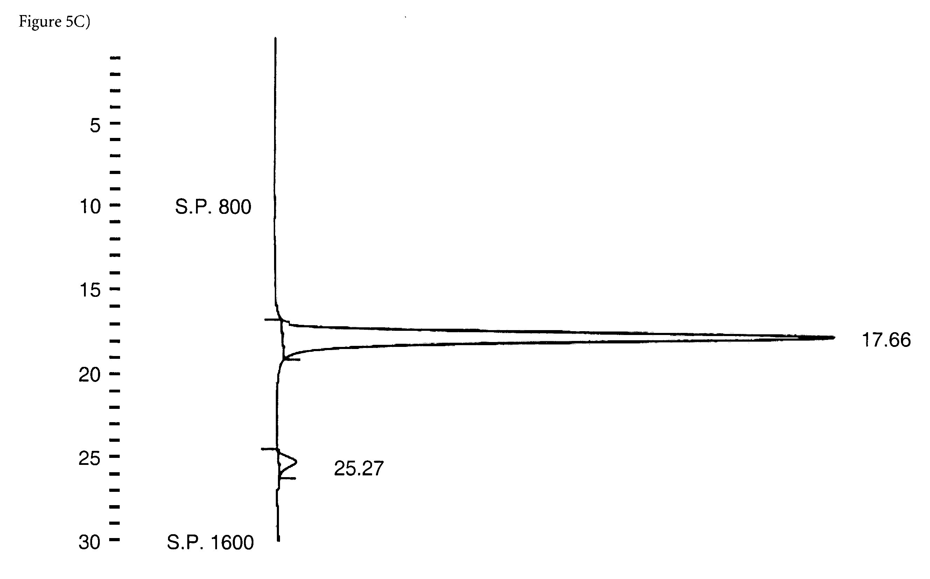

Figure 5 shows size exclusion chromatograms of A) molecular weight standard B) Mab-11 placebo and C) Mab-11. No aggregates or fragments were detectable. Equipment, working conditions and procedure were as described in Example 3.

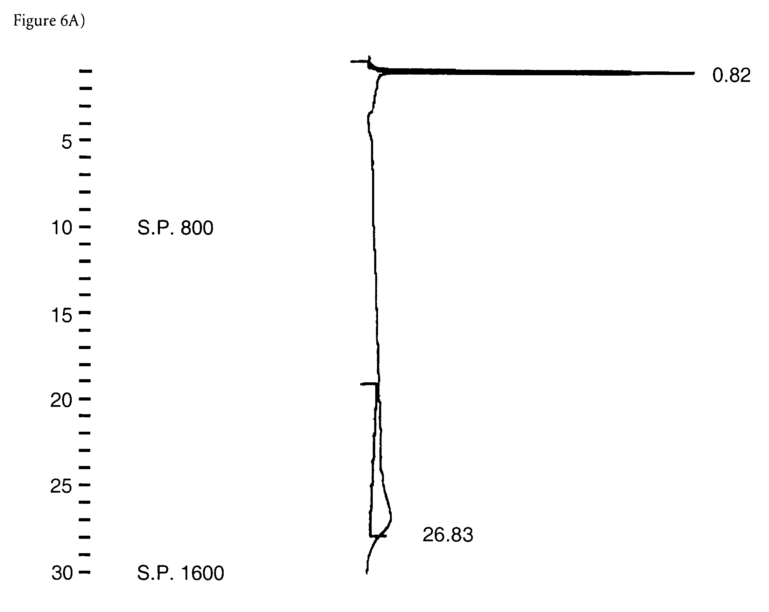

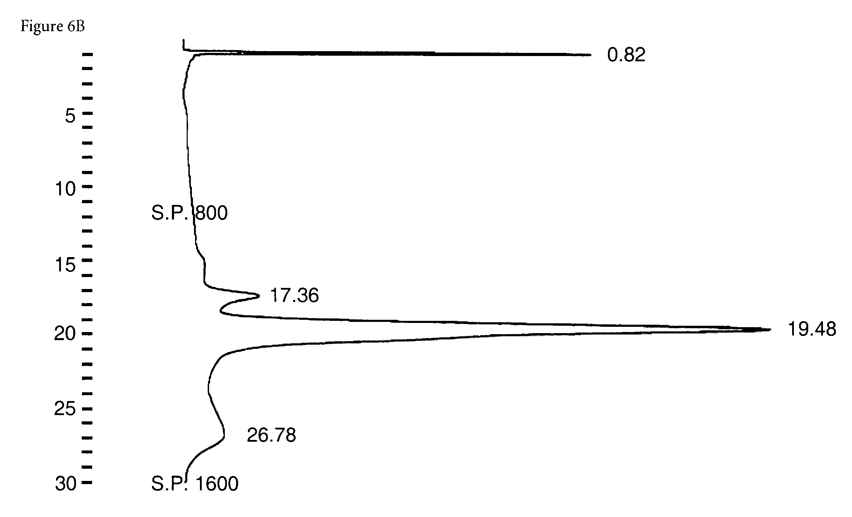

Figure 6 shows ion exchange chromatograms of Mab-11 placebo (left) and Mab-11 (right). Equipment, working conditions and procedure were as described in Example 3.

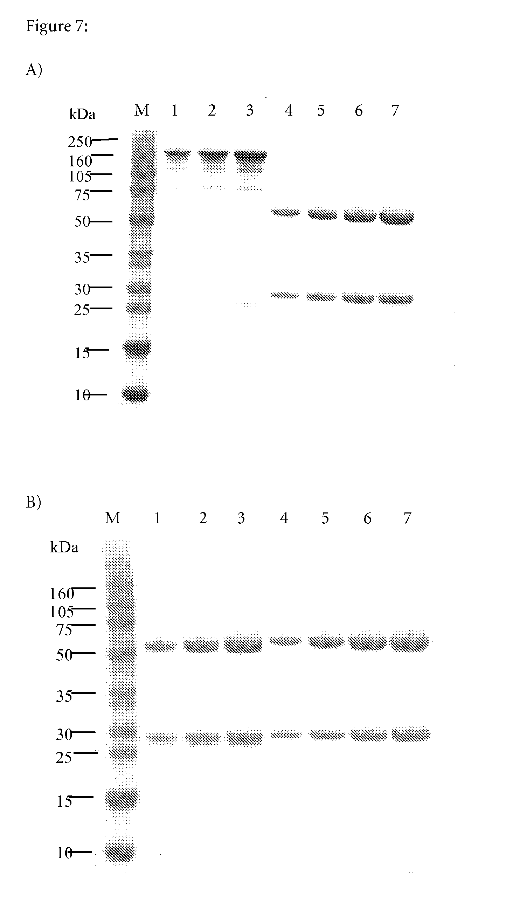

Figure 7 shows a representation of an SDS-PAGE analysis of Mab-11 and reduced/carboxymethylated Mab-11 (RA Mab-11) on 2-4% Bis-Tris gels run under non-reducing (A) and reducing conditions (B). Staining was performed with Coomassie Brilliant Blue Stain. M: Marker, lane 1 to 3: Mab-11, lane 4 to 7: RA-Mab-11.

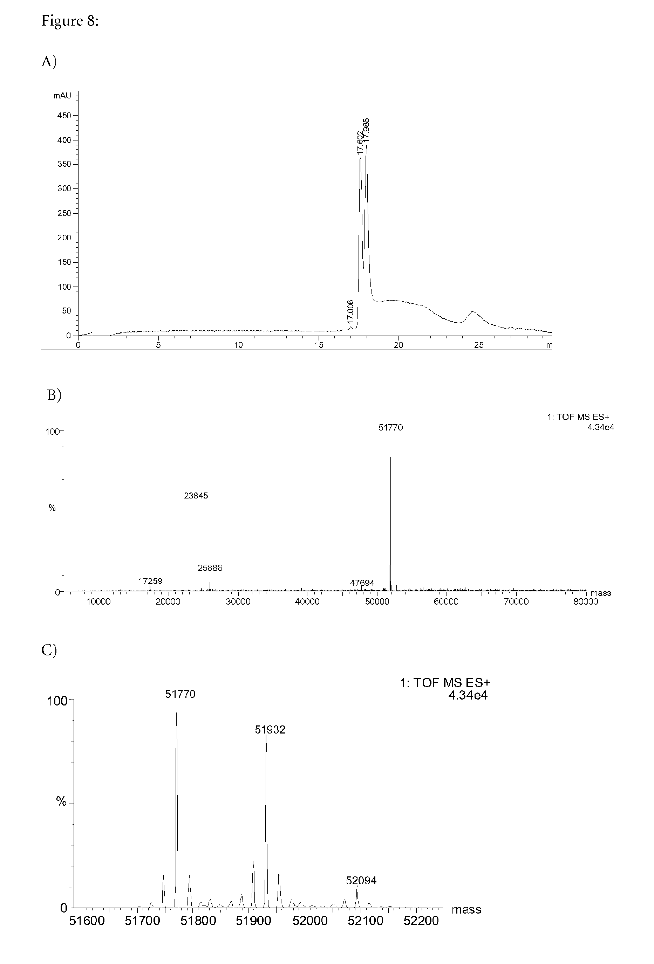

Figure 8 shows a representation of a LC/MS analysis of reduced/alkylated Mab-11 (RA Mab-11). A) HPLC chromatogram (C8 RP-HPLC, UV-trace, 214nm), the peaks represent the light and heavy chain; B) deconvoluted mass-spectra; C) zoomed region of the high molecular weight (HMW) mass peak in B). The detected masses are shown in Table 3.

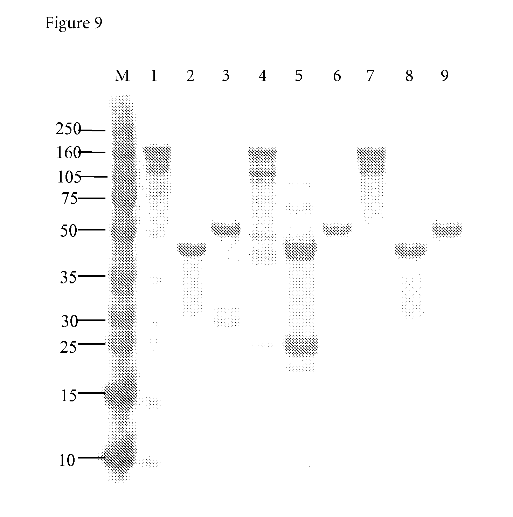

Figure 9 shows a SDS-PAGE of the full antibodies, purified Fab and Fc fragments prepared and isolated as described in example 5. Numbers on the left represent band positions (in kDa) of the molecular weight marker. Staining was performed wih Coomassie Brilliant Blue Stain. SDS-PAGE was performed under non-reducing conditions.
   M: Molecular weight marker, 1: Humira, full antibody, 2: Humira, Fab-fragment, 3: Humira, Fc fragment, 4: Synagis, full antibody, 5: Synagis, Fab fragment, 6: Synagis, Fc fragment, 7: Mab-11, full antibody, 8: Mab-11, Fab fragment, 9: Mab-11, Fc fragment. The antibodies and fragments were diluted in MES buffer and the loaded amount was 2µg for each sample.

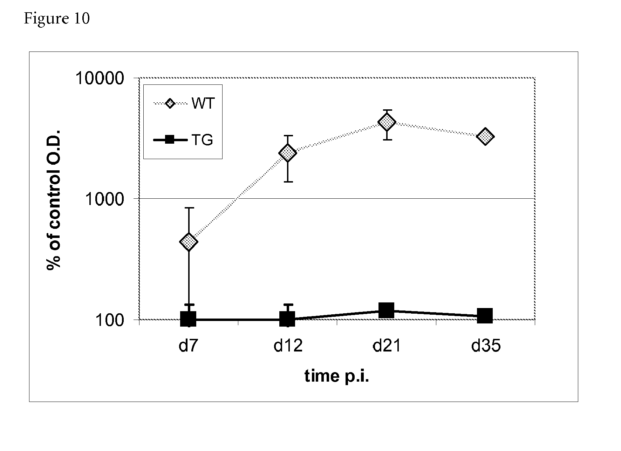

Figure 10 shows a graphical representation of an ELISA of anti-Mab-11 response on day 7, 12, 21 and 35 in the sera of wildtype (WT) and Mab-11 transgenic (TG) animals immunized with Mab-11 on day 0. The graphs show average values for immunizations of 5 WT and 5 TG mice, respectively. Results are expressed as multiples of O.D. of pre-immunization sera.

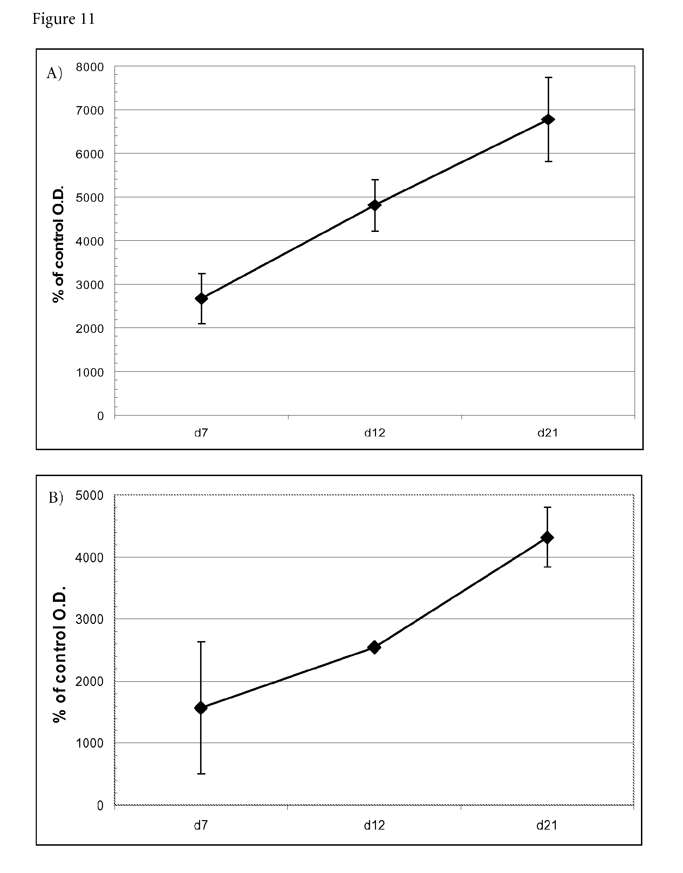

Figure 11 shows a graphical representation of ELISAs of anti-Humira response in the sera of (A) wild-type mice and (B) Mab-11 transgenic mice, expressed as multiples of O.D. of pre-immunization sera. 5 wild-type (WT) and 5 transgenic mice (TG) were immunized with Humira on day 0. On day 7, 12 and 21, anti-Humira antibody titers were assessed.

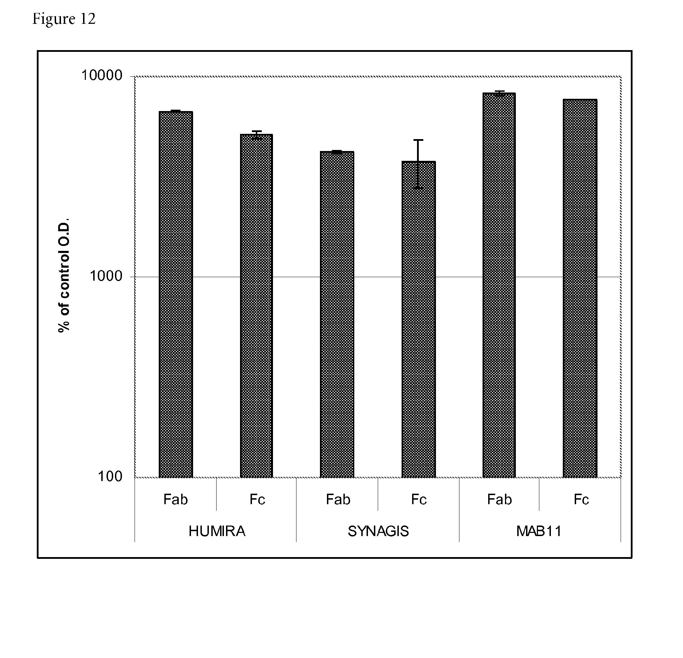

Figure 12 shows a graphical representation of an ELISA of sera reactivity to Fab- and Fc-fragments of Humira, Synagis and Mab-11 in a pool of sera of 5 wild-type mice 21 days after immunization with Humira. Binding is expressed as multiples of O.D. of control (wildtype pre-immunization) sera.

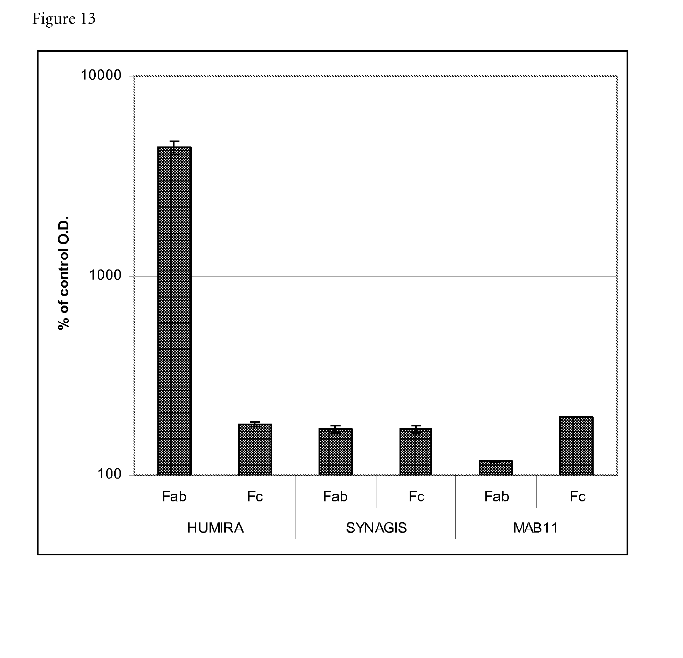

Figure 13 shows a graphical representation of an ELISA of sera reactivity to Fab- and Fc-fragments of Humira, Synagis and Mab-11 in a pool of sera of 5 Mab-11 transgenic mice 21 days after immunization with Humira. Binding is expressed as multiples of O.D. of control (wildtype pre-immunization) sera.

### Examples:

Commercially available reagents referred to in the examples were used according to manufacturer's instructions unless otherwise indicated.

### Example 1: Generation of mice transgenic for human immunoglobulin

### Generation of Mab-11 construct

A cDNA encoding an immunoglobulin (Ig) heavy chain (H) of the isotype γ1 (SEQ. ID. NO: 1) and a cDNA encoding a light chain (L) of the isotype κ (SEQ. ID. NO: 2) and with specificity for human Antibody peptide were used [Bardroff, M.e.a., Anti-amyloid beta antibodies and their use. EP03001759 EP, 2003]. This antibody anti-Abeta IgG1 is also referred to as Mab-11.

The cDNAs were amplified in a PCR reaction using the primers in Table 1. The 5 primers contain a SalI (or compatible XhoI) site and the 5' primers contain a BamHI (or compatible BglII) site for directed insertion into the pHSE3' vector [Pircher, H., et al., T cell tolerance to Mlsa encoded antigens in T cell receptor V beta 8.1 chain transgenic mice. Embo J, 1989. 8(3): p. 719-27.]. The PCR-amplified cDNAs were first enzymatically cut with both restriction enzymes SalI and BamHI, and then individually inserted into the corresponding sites of the vector pHSE3'. The expression of the Ig cDNAs in pHSE3' is driven by the murine promoter of the MHC class I gene H-2k and enhanced by the murine IgH gene enhancer located 3' of the cloned genes [Pircher, H., et al., T cell tolerance to Mlsa encoded antigens in T cell receptor V beta 8.1 chain transgenic mice. Embo J, 1989. 8(3): p. 719-27.]. This expression vector ensures high levels of production of the corresponding inserted gene products in T and B lymphocytes of the transgenic mice ([Pircher, H., et al., T cell tolerance to Mlsa encoded antigens in T cell receptor V beta 8.1 chain transgenic mice. Embo J, 1989. 8(3): p. 719-27.] and unpublished observations). The entire expression cassette, including (from 5' to 3'): the H-2k promoter, the inserted cDNA, the poly-A and splice sites and the Ig H gene enhancer element, was then excised from the vector by means of restriction enzyme cut with XhoI and agarose gel purification, and prepared in an adequate concentration for microinjection into fertilized mouse oocytes (2 µg/ml in 10 mM TrisHCl/0.1 mM EDTA, pH 7). The coding potential of the cDNA was confirmed by sequencing the entire cDNAs encoding the anti-Aβ Ig H and L genes (see figure 2 and 3).

**Table 1: Sequences of cloning primers.**

| Primer name | Sequence | Restriction site (in bold) | SEQ. ID |
|---|---|---|---|
| G1.11 Salfor (5'IgH) | 5'-ACG**TGTCGAC**GCCGCCACCATGAAACACCTG-3' | Sall (**GTCGAC**) | 3 |
| G1.11Bamrev (3'IgH) | 5·-ACG**TGGATCCT**CATTTACCCGGAGACAG-3' | BamHI (**GGATCC**) | 4 |
| K.11Xhofor (5'IgL) | 5'-ACG**TCTCGAG**GCCGCCACCATGGTGTTGCAG-3' | XhoI (**CTCGAG**) | 5 |
| K.11Bglrev (3'IgL) | 5'-ACG**TAGATCT**CTAACACTCTCCCCTGTTG-3' | BglII (**AGATCT**) | 6 |

### Generation of anti-AβIgG1 transgenic mice

Fertilized oocytes obtained from C57BL/6 female donors were microinjected with a 1:1 mixture of the purified XhoI fragments encoding for the IgH and L genes described in the previous section to obtain double transgenic animals. Pups born from these microinjected embryos were screened for the presence of the transgenes by amplifying genomic DNA prepared from tail biopsies with specific primers. The primers used are indicated in Table 2.

**Table 2: Primer sequences for detection of transgenes.**

| **PCR assay** | **Primers** | | **PCR fragment** | **SEQ. ID.** |
|---|---|---|---|---|
| Ig H gene | 5H2KP | 5'-ATGAATTCACAGTTTCACTTCTGCACC-3' | 660 bp | 7 |
| | G1.0501 | 5'-TGTACTCCTTGCCATTCAGC-3' | | 8 |
| Ig L gene | 5H2KP | 5'-ATGAATTCACAGTTTCACTTCTGCACC-3 | 660bp | 9 |
| | K.44 | 5'-GCTCATCAGATGGCGGGAAG-3' | | 10 |

The PCR reaction was performed using 1µl (about 100 ng) of total DNA obtained from the tail biopsy, in PCR reaction with the following conditions: 1 min at 90°, 30x [10 sec at 94°, 30 sec at 64°, 90 sec at 72°], 7 min at 72°. The PCR-amplified DNA fragments of about 660 bp were finally visualized in 1.5 % agarose gels separately for the transgenic IgH and L genes.

### Example 2: Phenotypic characterization of transgenic mice

### Serum analysis

Blood was obtained by tail vain puncture. Coagulation was performed overnight at room temperature. Serum was separated by centrifugation at 500xg for 10' and frozen at-20°C until further analysis.

To determine whether transgenic mice express fully human antibodies, an ELISA system was developed. Human antibodies were captured using a polyclonal goat-anti-human kappa-chain specific antibody (Sigma K 3502). Detection was performed with a monoclonal mouse-anti-human gamma-chain specific antibody coupled to peroxidase (POD, Sigma A 0170). As shown in figure 4, transgenic mice express fully human immunoglobulin.

The response against a closely related antigen was assessed by immunization with the human IgG1 antibody HUMIRA (Abbott). 10µg of HUMIRA were emulsified in 200µl of Rehydragel HPA (Reheis). Animals were immunized intraperitonial (i.p.) on day 0, blood was drawn by tail vain puncture on days 7, 12, 21 and 35, serum was prepared, and anti-HUMIRA titers were assessed by ELISA, using HUMIRA for capture by coating to maxisorp plates (Nunc) and detecting anti-HUMIRA antibodies by anti-mouse IgG (BD Pharmigen). As shown in figure 11, both wild-type and transgenic mice mount a response against the closely related human IgG1 antibody HUMIRA.

### Example 3: Production, purification and characterization of Mab-11 (also referred to as anti-Abeta IgG1)

Mab-11 was produced under serum-free conditions in Chinese hamster ovary cells transfected with cDNA encoding an IgG1 of the same sequence as the transgene outlined in Example 1.

### Isolation and purification of Mab-11 from fermentation supernatant

All procedures were performed under endotoxin-free conditions by using tempered glassware only, sanitization of all equipment including columns was done with 0.5M NaOH and sterile filtration of all buffers (0.22µm). Only fresh gel material was used.

As first purification step, Protein A affinity chromatography was performed using Mab Select gel (Amersham). After a pre-run, the column was equilibrated with 25 mM Tris/HCl; 25 mM NaCl, 5 mM EDTA pH 7.1, and filtered supernatant of the CHO cell culture was loaded onto the gel. Protein A-bound antibody was eluted with 100mM HAc pH 2.9. For virus inactivation, the eluate was adjusted to pH ≤ 3.6 with HAc and then incubated for 15 minutes at RT, then adjusted with 1M Tris to pH 4.0. As second step of purification, ion exchange chromatography (cation exchange chromatography) using SP-Toyopearl 650M (Tosoh) as matrix. After a pre-run, eluate fractions of step one were loaded onto the gel, equilibrated with buffer A (50 mM HAc, pH 5.0), then a gradient elution from 0 to 100% buffer B (50mM HAc, 1M NaCl pH 5.0) was performed. Eluting protein fractions were collected, concentrated by ultrafiltration, adjusted to pH 7.5 and analyzed by IEX and SEC HPLC. As third purification step, size exclusion chromatography (flow-through anion exchange chromatography) was performed using Q-Sepharose FF gel (Biorad) as stationary and 25mM Tris/HCl, 80 mM Na-acetate, pH 7.5 as mobile phase. The effluent was fractionated according to the UV signal. Exchange of buffer to Mab-11 placebo (buffer without Mab-11) containing 20 mM Histidine/140 mM NaCl, pH 5.5 and adjustment of concentration was done in an Amicon stirred cell (Amicon) using a 10 kDa membrane. The solution was finally filtered over a 0.22 µm Millex-GV sterile filter (Millipore) and stored in aliquots at -80°C.

### Characterization of anti-Abeta-IgG1 (Mab-11)

Integrity and heterogeneity of purified Mab-11 IgG1 protein was assessed by SDS-PAGE, Size Exclusion- and Ion Exchange Chromatography and confirmation of the primary sequence was done by LC/MS analysis of reduced and carboxymethylated Mab-11-antibody as described below.

### Size exclusion chromatography

Samples of purified Mab-11 were analyzed by size exclusion chromatography by using a Jasco PU-980 HPLC system. The samples were chromatographed on a TSK-Gel G3000SWXL, 7.8 x 300 mm, 5 µm column (Tosho Biosciences) with 0.2M K2HPO4, 0.25M KCl, pH 7.0 as mobile phase. The flow rate was set at 0.5 ml/min. The absorbance was monitored at 220 nm using a Jasco UV-975 detector connected to a Merck-Hitachi D-2500 recording system. The column was equilibrated until a stable baseline was obtained. A sequence was injected corresponding gel filtration standard (BioRad, 151-1901, including 670 kD bovine thyroglobulin, 158 kD bovine IgG, 44 kD OVA, 17 kD eq. myoglobin, 1.35 kD vit.B12), Mab-11 placebo (negative control: buffer without Mab-11), Mab-11 sample. Injected amount corresponded to approximately 50 µg of sample. Representative size exclusion chromatograms are shown in figure 5. Symmetrical peak at retention time corresponded to 155-160 kDa. No aggregates or fragments were detectable.

### Ion exchange chromatography

Samples of purified Mab-11 were analyzed by ion exchange chromatography using the Jasco PU-980 HPLC system. The samples were chromatographed on a Mono S 5/50 GL column (Amersham Biosciences) by using a gradient from 0 % B to 52 % B in 20 min. (Mobile Phase A: 50mM malonic acid/malonate in water, pH5.3; Mobile Phase B: 1 M Na-acetate in Mobile Phase A, pH5.3). The flow rate was set at 1 ml/min. The absorbance was monitored at 280 nm using a Jasco UV-975 detector connected to a Merck-Hitachi D-2500 recording system. The column was equilibrated with Mobile Phase A until a stable baseline was obtained. A sequence was injected corresponding Mab-11 placebo and Mab-11 sample. Injected amount of Mab-11 corresponded to approximately 50 µg. Representative ion exchange chromatograms (IEC) are shown in figure 6. Result: > 95% of Mab-11 sample elutes as single peak with non-resolved shoulder at higher retention time. About 5% elute with shorter retention time.

### Sodium dodecyl sulfate polyacrylamide gel electrophoresis

Samples of purified Mab-11 and reduced/carboxymethylated Mab-11 (RA Mab-11, preparation see below) were analyzed by SDS-PAGE using a Xcell Mini-Cell II Gel system (Invitrogen). Pre-diluted samples were mixed with reducing or non-reducing sample buffer to concentrations of 2-8 µg per 20 µL. Reducing sample buffer was prepared by mixing NuPAGE LDS sample buffer (Invitrogen) with NuPAGE reducing agent (Invitrogen) according manufacturer's advice. Samples in reducing sample buffer were incubated for 10 minutes at 70°C. Samples and standard were loaded on a NuPAGE 4-12 % Bis-Tris Gel, 10 well-comb, 1.0 mm thickness (Invitrogen, #NP0301BOX). As standard, Mark 12TM Molecular Weight Marker (Invitrogen, #LC5677) covering a range of 200-2.5kDa was used. The gels were run at 200 V for 1h with NuPAGE MOPS SDS running buffer (Invitrogen). Gels were stained over night with StainEase Gel staining tray (Invitrogen), scanned and analyzed by densitometry. Protein concentrations were calculated referring to a standard sample curve obtained from the reference standard run in the same gel. Results: SDS-PAGE under non-reducing conditions: for full Mab-11, bands corresponding to the expected molecular weight of IgG; for RA MAb-11, two bands corresponding to molecular weights of IgG1 H- and L-chains. No full or partially reduced Mab-11 detectable. SDS-PAGE under reducing conditions: for both full Mab-11 and RA Mab-11, two bands corresponding to molecular weights of IgG1 H- and L-chains. No aggregates or fragments detectable (see figure 7).

### Mass spectrometry

Confirmation of the primary sequence was done by LC/MS analysis. Reduced and carboxymethylated Mab-11-antibody was prepared as described in Lundell and Schreitmüller (Sample preparation for peptide mapping--A pharmaceutical quality-control perspective. Anal Biochem. 1999 Jan 1;266(1):31-47) and were subjected to an analytical RP-HPLC analysis on an Agilent Poroshell C8-reversed-phase (0.5 x 75 mm) colum using a gradient system (A: water 0.1 % formic acid, B: acetonitrile 0.1 % formic acid). The chromatography stream was subsequently subjected to the ESI ion source of an ESI-Q-TOF mass spectrometer (Micromass/Waters Q-TOF Ultima, Manchester, UK) in the positive mode with lockspray mass correction. Protein spectra were deconvoluted with the Masslynx MaxEnt1 module.

LC/MS analysis of RA-Mab-11 is shown in Figure 8, observed and calculated masses are assigned in Table 3.

**Table 3: LC/MS analysis of RA-Mab-11, assignments of observed masses (see Figure 8). L-chain = light chain, H-chain = heavy chain, G₀, G₁, G₂ = glycosylation on H-chain; Pyro-Glu = Amino acid sequence comprises a pyroglutamate, - Lys = one lysine is missing in the amino acid sequence of the H-chain.**

| Observed mass, [M+H]⁺ | Assignment (theoretical mass [M+H]⁺) |
|---|---|
| 23845 Da | Mab-11; L chain, reduced and carboxymethylated (23844 Da) |
| 51770 Da | Mab-11; H chain-G₀, Pyro-Glu, - Lys, reduced and carboxymethylated (51770 Da) |
| 51932 Da | Mab-11; H chain-G₁, Pyro-Glu, - Lys, reduced and carboxymethylated (51932 Da) |
| 52094 Da | Mab-11; H chain-G₂, Pyro-Glu, - Lys, reduced and carboxymethylated (52094 Da) |

The detected masses match with the theoretically expected masses for reduced/carboxymethylated H- and L-chain of an antibody with Mab-11 primary sequence with one pyro-Glu and with one Lys missing in the H-chain. The H-chain is mainly G₀- and G₁ glycosylated, G₂ was found only to a minor extend.

### Example 4: Immunological assessment of tolerance.

Transgenic and wild-type littermate control mice (N = 5/each) were immunized i.p. with 10 µg recombinant Mab-11 emulsified in 200 µl of Rehydragel-HPA (Reheis). Blood was obtained by tail vain puncture on days 7, 12, 21 and 35. Serum was prepared by coagulation as described above, serum anti-Mab-11 titers were measured in ELISA. 0.2 ug/well Mab-11 were coated O/N at RT on maxisorp plates (Nunc). After washing, wells were blocked with PBS/0.1% (v/v) Tween-20/0.1% (w/v) BSA and 1:100 diluted sera were added and incubated 1h under orbital shaking. Binding of antibodies detected by APK-labeled anti-mouse IgG (BD Pharmingen) in 1:100 dilution according the manufacturer's advice.

ELISA analysis revealed the generation of a robust immune response against Mab-11 in the wild-type animals, while hIgG1-transgenic animals were unable to mount an immune response to recombinant Mab-11 (figure 10). Thus, Mab-11 transgenic mice are tolerant towards the product of their transgene in form of exogenously supplied antibody.

### Example 5: Generation and isolation of Fab- and Fc-fragments of Humira, Synagis and Mab-11

The monoclonal antibodies Humira^{®}, Synagis^{®} (Palivizumab, ABBOTT AG, #2422260A) and Mab-11 were digested with Papain and the generated Fab- and Fc fragments isolated by ion exchange chromatography (IEC). In brief, the formulation buffers of the antibodies were changed to 0.1M Tris, 4mM EDTA, 1mM Cystein pH 7.4 by using sephadexTM G-25M PD10 columns (Amersham Biosience) and diluted to a concentration of 3-5 mg/ml. Papain (Roche Diagnostics) was added to a final concentration of 0.01 mg/mL. After 2h incubation at 37°C, the buffer was changed to 20mM L-Histidine pH 5.5 by 10kDa ultrafiltration using Amicon Ultra Centrifugal Filter Devices (Millipore). Fab- and Fc fragments were isolated by preparative IEC using a PL-SCX 1000A, 8um, 4.6 x 150 mm (Polymer Labs) or a Mono STM 5/50 GL column (Amersham Biosciences) and a gradient of 50 mM 3-Morpholinpropanesulfonuic acid (MOPS)(Applichem) pH 6.7 to 1 M Na-acetate in 50 mM MOPS, pH 7.0; or a gradient of 10mM 2-(N-Morpholino) ethane sulfonic acid (MES) pH 6.0 to 10mM MES, 0.2 M NaCl, pH 6.0, repectively.

Protein containing fractions were collected and buffer exchanged to 20 mM L-Histidine, 140 mM NaCl, 0.01% Tween pH 5.5 by 10 kDa ultra-filtration (Amicon Ultra, see above).

The concentrated fractions were characterized by SDS-PAGE under non-reducing conditions as described in example 3. The result is shown in Figure 9.

### Example 6: Generation of anti-idiotypic antibodies

Humira^{®} (Adalimumab, ABBOTT AG, #04H-640-E694-1) was used as an example for an idiotypic antibody of the same isotype as Mab-11. Humira is a TNF-specific therapeutic monoclonal human antibody for treatment of rheumatoid arthritis. Both Mab-11 and Humira are IgG1 antibodies with identical or nearly identical primary sequence in their Fc parts and constant Fab regions. 10 µg of HUMIRA were emulsified in 200 µl of Rehydragel HPA (Reheis). Animals were immunized intraperitonial (i.p.) on day 0, blood was drawn by tail vain puncture on days 7, 12 and 21, serum was prepared, and anti-HUMIRA titers were assessed by ELISA. HUMIRA was used for capture by coating to maxisorp plates (Nunc), detection was performed by anti-mouse IgG (BD Pharmigen) as described above. As shown in figure 11, both wild-type (A) and transgenic mice (B) generate a robust response against HUMIRA.

### Example 7: Assessment of cross reactivity of anti-Humira sera against idiotypic antibodies

To assess the specificity of anti-Humira antibodies in sera of Humira-immunized WT and TG mice, ELISAs were performed using Humira, Mab-11 and Synagis as examples for idiotypic human IgG1 with identical or nearly identical primary sequence in their Fc-parts and constant Fab-regions.

Synagis^{®} (Palivizumab, ABBOTT AG, #2422260A) is a monoclonal humanized IgG1 used for treatment of RSV infection.

Fab and Fc fragments of Humira, Mab-11 and Synagis were prepared and isolated as described in example 5, and coated O/N at RT on maxisorp plates (Nunc) at 0.2 µg/well. After washing, wells were blocked with PBS/0.1% (v/v) Tween-20/0.1% (w/v) BSA. 1:100 diluted pools of sera of five transgenic and five wild-type mice immunized with Humira were added and incubated 1h under orbital shaking.

Binding of antibodies detected by APK-labeled anti-mouse IgG (BD Pharmingen) in 1:100 dilution according the manufacturer's advice.

ELISA analysis revealed strong differences in the binding of sera antibodies to the tested antigens.

For the wild-type animals, the generation of a robust immune response against either Fab- and Fc fragments of all antibodies tested was observed. Thus, the immune response elicited in the wild-type animals is directed predominantly against constant parts of human IgG1, explaining the observed strong cross-recognition of either Fab and Fc parts of all antibodies tested (Figure 12).

In contrast, for Mab-11-transgenic mice immunized with Humira, a robust antibody response against the Fab part of Humira was detected, whereas binding to Humira Fc and Fab, and Fc parts of all other idiotypic antibodies tested was at background level (Figure 13).

Therefore, it can be concluded that the animals transgenic for the human antibody Mab-11 mounted an immune response predominantly against the variable, idiotype-specific parts of the idiotypic antibody used for immunization.

It has been shown that the IgG1 transgenic mice presented here are tolerant to the IgG1 antibody expressed transgenically, but develop a robust immune response when challenged with different human antibody of the same IgG1 isotype. The elicited immune response is exclusively directed towards the V region of the human IgG1 molecule used for challenge, whereas the Fc region is exquisitely ignored. This property of the human anti-Abeta IgG1 transgenic mouse makes it an invaluable tool for the rapid and efficient generation of anti-idiotypic monoclonal antibodies.

## Claims

1. Use of a transgenic non-human animal for generating anti-idiotypic antibodies against an antibody of interest, wherein said non-human animal is transgenic for an exogenous antibody and wherein the antibody of interest has the same isotype as the exogenous antibody.

2. The use according to claim 1 wherein the exogenous antibody and the antibody of interest are human or humanized antibodies.

3. The use according to any one of the claims 1 or 2 wherein the exogenous antibody and the antibody of interest are IgG antibodies.

4. A method for generating anti-idiotypic antibodies against an antibody of interest comprising:
a) creating a non-human animal transgenic for an exogenous antibody
b) inducing an immune response in said transgenic non-human animal against an antibody of interest, whereby the antibody of interest has the same isotype as the exogenous antibody, and
c) generating antibodies directed against the idiotypic part of the antibody of interest.

5. The method according to claim 4, wherein the method comprises additionally step d) isolating the anti-idiotypic antibodies.

6. The method according to claim 4 or 5 wherein the exogenous antibody and the antibody of interest are human or humanized antibodies.

7. The method according to any one of the claims 4 to 6 wherein the exogenous antibody and the antibody of interest are IgG antibodies.

8. Methods and uses substantially as herein before described especially with reference to the foregoing examples


SEQUENCE LISTING

<110>  F. Hoffmann-La Roche AG

<120>  Method for generating idiotypic antibodies

<130>  23856

<160>  10

<170>  PatentIn version 3.3

<210>  1
<211>  1562
<212>  DNA
<213>  Homo sapiens

<400>  1
gcgccaccat gaaacacctg tggttcttcc tcctgctggt ggcagctccc agatgggtcc      60

tgtcccaggt ggaattggtg gaaagcggcg gcggcctggt gcaaccgggc ggcagcctgc     120

gtctgagctg cgcggcctcc ggatttacct ttagcagcta tgcgatgagc tgggtgcgcc     180

aagcccctgg gaagggtcta gagtgggtga gcggtattaa tgctgctggt tttcgtactt     240

attatgctga ttctgttaag ggtcgtttta ccatttcacg tgataattcg aaaaacaccc     300

tgtatctgca aatgaacagc ctgcgtgcgg aagatacggc cgtgtattat tgcgcgcgtg     360

gtaagggtaa tactcataag ccttatggtt atgttcgtta ttttgatgtt tggggccaag     420

gcaccctggt gacggttagc tcagcctcca ccaagggccc atcggtcttc cccctggcac     480

cctcctccaa gagcacctct gggggcacag cagccctggg ctgcctggtc aaggactact     540

tccccgaacc ggtgacggtg tcgtggaact caggcgccct gaccagcggc gtgcacacct     600

tcccggctgt cctacagtcc tcaggactct actccctcag cagcgtggtg accgtgccct     660

ccagcagctt gggcacccag acctacatct gcaacgtgaa tcacaagccc agcaacacca     720

aggtggacaa gaaagttgag cccaaatctt gtgacaaaac tcacacatgc ccaccgtgcc     780

cagcacctga actcctgggg ggaccgtcag tcttcctctt ccccccaaaa cccaaggaca     840

ccctcatgat ctcccggacc cctgaggtca catgcgtggt ggtggacgtg agccacgaag     900

accctgaggt caagttcaac tggtacgtgg acggcgtgga ggtgcataat gccaagacaa     960

agccgcggga ggagcagtac aacagcacgt accgtgtggt cagcgtcctc accgtcctgc    1020

accaggactg gctgaatggc aaggagtaca agtgcaaggt ctccaacaaa gccctcccag    1080

cccccatcga gaaaaccatc tccaaagcca aagggcagcc ccgagaacca caggtgtaca    1140

ccctgccccc atcccgggat gagctgacca agaaccaggt cagcctgacc tgcctggtca    1200

aaggcttcta tcccagcgac atcgccgtgg agtgggagag caatgggcag ccggagaaca    1260

actacaagac cacgcctccc gtgctggact ccgacggctc cttcttcctc tacagcaagc    1320

tcaccgtgga caagagcagg tggcagcagg ggaacgtctt ctcatgctcc gtgatgcatg    1380

aggctctgca caaccactac acacagaaga gcctctccct gtctccgggt aaatgagtgc    1440

cacggccggc aagcccccgc tccccaggct ctcggggtcg cgcgaggatg cttggcacgt    1500

accccgtgta catacttccc aggcacccag catggaaata aagcacccag cgcttccctg    1560

gg                                                                    1562

<210>  2
<211>  715
<212>  DNA
<213>  Homo sapiens

<400>  2
cgccaccatg gtgttgcaga cccaggtctt catttctctg ttgctctgga tctctggtgc      60

ctacggggat atcgtgctga cccagagccc ggcgaccctg agcctgtctc cgggcgaacg     120

tgcgaccctg agctgcagag cgagccagta tgttgatcgt acttatctgg cgtggtacca     180

gcagaaacca ggtcaagcac cgcgtctatt aatttatggc gcgagcagcc gtgcaactgg     240

ggtcccggcg cgttttagcg gctctggatc cggcacggat tttaccctga ccattagcag     300

cctggaacct gaagactttg cgacttatta ttgccagcag atttattctt ttcctcatac     360

ctttggccag ggtacgaaag ttgaaattaa acgtacggtg gctgcaccat ctgtcttcat     420

cttcccgcca tctgatgagc agttgaaatc tggaactgcc tctgttgtgt gcctgctgaa     480

taacttctat cccagagagg ccaaagtaca gtggaaggtg gataacgccc tccaatcggg     540

taactcccag gagagtgtca cagagcagga cagcaaggac agcacctaca gcctcagcag     600

caccctgacg ctgagcaaag cagactacga gaaacacaaa gtctacgcct gcgaagtcac     660

ccatcagggc ctgagctcgc ccgtcacaaa gagcttcaac aggggagagt gttag          715

<210>  3
<211>  31
<212>  DNA
<213>  Artificial

<220>
<223>  Description of artificial sequence: G1.11Salfor, primer for 5´end
       of the heavy chain of human anti-Abeta IgG1

<400>  3
acgtgtcgac gccgccacca tgaaacacct g                                     31

<210>  4
<211>  28
<212>  DNA
<213>  Artificial

<220>
<223>  description of artificial sequence: G1.11Bamrev, primer for 3'
       end of heavy chain of human anti-Abeta IgG1

<400>  4
acgtggatcc tcatttaccc ggagacag                                         28

<210>  5
<211>  31
<212>  DNA
<213>  Artificial

<220>
<223>  Description of artificial sequence: K.11Xhofor, primer for 5' end
       of the light chain of human anti-Abeta IgG1

<400>  5

acgtctcgag gccgccacca tggtgttgca g                                    31

<210>  6
<211>  29
<212>  DNA
<213>  Artificial

<220>
<223>  Description of artificial sequence: K.11Bglrev, primer for 3' end
       of light chain of the human anti-Abeta IgG1

<400>  6
acgtagatct ctaacactct cccctgttg                                      29

<210>  7
<211>  27
<212>  DNA
<213>  Artificial

<220>
<223>  Description of artificial sequence: 5H2KP, PCR primer for heavy
       chain gene of human anti-Abeta IgG1

<400>  7
atgaattcac agtttcactt ctgcacc                                        27

<210>  8
<211>  20
<212>  DNA
<213>  Artificial

<220>
<223>  Description of artificial sequence: G1.0501, PCR primer for heavy
       chain gene of human anti-Abeta IgG1

<400>  8
tgtactcctt gccattcagc                                                20

<210>  9
<211>  27
<212>  DNA
<213>  Artificial

<220>
<223>  Description of artificial sequence: 5H2KP, PCR primer for light
       chain gene of human anti-Abeta IgG1

<400>  9
atgaattcac agtttcactt ctgcacc                                        27

<210>  10
<211>  20
<212>  DNA
<213>  Artificial

<220>
<223>  Description of artificial sequence: K.44, PCR primer for light
       chain gene of human anti-Abeta IgG1

<400>  10
gctcatcaga tggcgggaag                                                20